# EUROPEAN PATENT APPLICATION

(11) **EP 4 826 727 A1**
(43) Date of publication of application: **21.10.2026**
(21) Application number: 26171646.8
(22) Date of filing: 10.04.2026
(51) Int. Cl.: G16H 10/60, G16H 40/20, G06F 40/174, G06Q 10/10, G16H 10/20

(54) **ELECTRONIC CONSENT DOCUMENT SYSTEM**

(30) Priority: 15.04.2025 JP 2025066654
(71) Applicant: KYOCERA Document Solutions Inc., Osaka-shi, Osaka 540-8585 (JP)
(72) Inventor: FUJIMORI, Taiki, Osaka, 540-0004 (JP); HARADA, Eriko, Osaka, 540-0004 (JP); HIRANO, Masayoshi, Osaka, 540-0004 (JP); NOGAWA, Hiroaki, Osaka, 540-0004 (JP)
(74) Representative: Winter, Brandl - Partnerschaft mbB

(57) **Abstract**

A terminal apparatus displays an explanation document image of a specific matter, displays an electronic consent form of the specific matter after displaying the explanation document image, acquires signature data, after detecting the signature operation, displays an electronic consent document based on electronic consent document data that includes the signature data, and transmits the electronic consent document data to the management server after detecting a predetermined confirmation operation. Further, the terminal apparatus detects an additionally-writing operation performed by a healthcare worker onto the explanation document image and generates an additionally-writing image generates a signature image based on the signature operation, and includes the explanation document image, the additionally-writing image, the electronic consent form, and the signature image into the electronic consent document data, such that the additionally-writing image and the signature image are separated from each other.

## Description

### BACKGROUND

### 1. FIELD OF THE PRESENT INVENTION

The present invention relates to an electronic consent document system.

### 2. DESCRIPTION OF THE RELATED ART

In an information processing system, consent form data is displayed on a terminal apparatus of a patient, and when a signature of the patient is electronically inputted to the consent form data, the consent form data that the input information such as the signature has been applied is displayed on the terminal apparatus, and thereafter, pressing down a "consent" button causes the signature process to be finished, and thereafter, the consent form data is submitted to a server.

However, in the aforementioned information processing system, inputting the electronic signature and pressing down the "consent" button immediately complete the consent form, and therefore, at a later date, doubts on whether the patient's consent has been obtained or not may arise in the patient side or a third party. In other words, due to wrong memory, suspicions of falsification or the like, the patient or a third party may not agree the fact that the patient consented.

### SUMMARY

An electronic consent document system according to the present invention includes a management server connected to an in-hospital network, and a terminal apparatus connected to the in-hospital network. The terminal apparatus (a) displays an explanation document image of a specific matter, (b) displays an electronic consent form of the specific matter after displaying the explanation document image, (c) acquires signature data on the basis of a signature operation to the electronic consent form, (d) after detecting the signature operation, displays an electronic consent document based on electronic consent document data that includes the signature data corresponding to the electronic consent form, and (e) transmits the electronic consent document data to the management server after detecting a predetermined confirmation operation. Further, the terminal apparatus (a) detects an additionally-writing operation performed by a healthcare worker onto the explanation document image and generates an additionally-writing image based on the additionally-writing operation, (b) generates a signature image based on the signature operation, and (c) includes the explanation document image, the additionally-writing image, the electronic consent form, and the signature image into the electronic consent document data and transmits the electronic consent document data to the management server. Here, the additionally-writing image and the signature image are included into the electronic consent document data such that the additionally-writing image and the signature image are separated from each other.

These and other objects, features and advantages of the present invention will become more apparent upon reading of the following detailed description along with the accompanied drawings.

### BRIEF DESCRIPTION OF THE DRAWINGS

Fig. 1 shows a block diagram that indicates a configuration of an electronic consent document system according to an embodiment of the present invention;
Fig. 2 shows a diagram that indicates a display example of an explanation document image;
Fig. 3 shows a diagram that indicates a display example of an electronic consent form after date input and handwriting signature;
Fig. 4 shows a diagram that indicates a display example of an electronic consent form (confirmation image);
Fig. 5 shows a diagram that indicates an example of the printed electronic consent document; and
Fig. 6 shows a sequence diagram that explains a behavior of the electronic consent document system shown in Fig. 1.

### DETAILED DESCRIPTION

Hereinafter, an embodiment according to the present invention will be explained with reference to drawings.

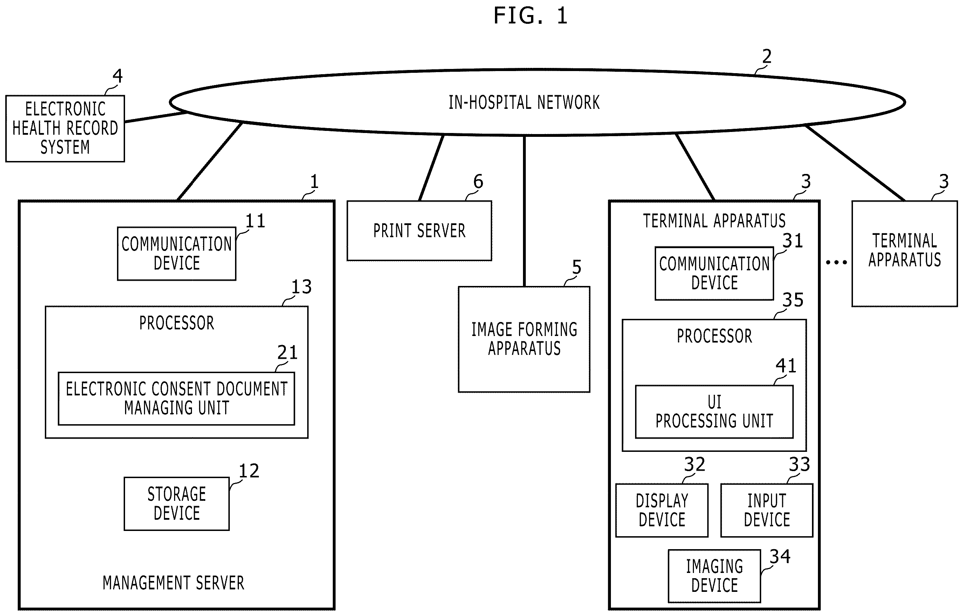

Fig. 1 shows a block diagram that indicates a configuration of an electronic consent document system according to an embodiment of the present invention. In the electronic consent document system shown in Fig. 1, a management server 1 is connected to an in-hospital network 2, and to the in-hospital network 2, one or plural terminal apparatuses 3, an electronic health record system 4, an image forming apparatus 5, and a print server 6 are connected.

The in-hospital network 2 is a local area network in a hospital, a clinic or the like, and the management server 1 is an on-premise server apparatus that includes a computer. Each of the terminal apparatuses 3 is connected to the in-hospital network 2 through wireless or wired communication, is carried by a healthcare worker, and presents an explanation document, an electronic consent form, and the like when explaining a specific matter (surgery or the like) to a patient. The terminal apparatus 3 is a personal computer or a portable terminal such as a tablet terminal or a smart phone.

The electronic health record system 4 is a system that manages an electronic health record with an on-premise server apparatus, and enables the terminal apparatus connected to the in-hospital network 2 to perform generation of a new electronic health record, editing of an electronic health record, viewing of an electronic health record and the like.

The image forming apparatus 5 is an apparatus such as printer or multi function peripheral, that has a printing function. Here, the image forming apparatus 5 acquires a print request (print image data, print settings and the like) from the print server 6 through the in-hospital network 2, but alternatively, the image forming apparatus 5 may be directly connected to the print server 6 with a peripheral device interface (USB or the like) and may acquire a print request from the print server 6 not through the in-hospital network 2. The print server 6 receives and spools a print request from the management server 1 and transmits the print request to the image forming apparatus 5.

As mentioned, this electronic consent document system is an on-premise system, and therefore, easily cooperates with the existing on-premise electronic health record system 4.

Further, the management server 1 includes a communication device 11, a storage device 12, and a processor 13. The communication device 11 is a device such as a network interface connected to the in-hospital network 2. The storage device 12 is a non-volatile storage device such as flash memory, and stores data and/or a program used by the processor 13. The processor 13 includes a computer and the computer includes a CPU (Central Processing Unit), a ROM (Read Only Memory), a RAM (Random Access Memory) and the like, loads a program stored in the ROM or the storage device 12 to the RAM, executes the program with the CPU, and thereby acts as sorts of processing units. Here, the computer act as an electronic consent document managing unit 21.

Meanwhile, each of the terminal apparatuses 3 includes a communication device 31, a display device 32, an input device 33, an imaging device 34, and a processor 35. The communication device 31 is a wired or wireless network interface or the like connected to the in-hospital network 2.

The display device 32 includes a liquid crystal display panel or the like and displays a view screen, an operation screen and the like. The input device 33 includes a touch panel, a hard key and/or the like and detects a user operation. For example, a touch panel as the input device 33 is arranged on a display panel of the display device 32, and the display device 32 displays an image of a soft key at predetermined area and the input device 33 detects a user operation to the area and thereby the soft key is formed.

The imaging device 34 includes an imaging sensor, and takes and generates an image (image data) of a scene in front of a housing that the display device 32 and the input device 33 are arranged (e.g. an image of a user who operates this terminal apparatus 3 or the like).

The processor 35 includes a computer and the computer includes a CPU, a ROM, a RAM and the like, and loads a program stored in the ROM or the like to the RAM and executes the program with the CPU and thereby acts as processing units. Here, the computer acts as a user interface processing unit 41.

Further, the user interface processing unit 41 in the terminal apparatus 3 displays an electronic consent form of a specific matter (e.g. medical procedure such as surgery) on the display device 32, acquires signature data using the input device 33 on the basis of a signature operation to the electronic consent form, and transmits using the communication device 31 electronic consent document data that includes the signature data corresponding to the electronic consent form.

The electronic consent document data may be a data set that includes the signature data and the electronic consent form or the electronic consent document data may be raster image data obtained by synthesizing the signature data and the electronic consent form.

Meanwhile, the electronic consent document managing unit 21 in the management server 1 receives the electronic consent document data from the terminal apparatus 3 through the in-hospital network 2 using the communication device 11, and when receiving the electronic consent document data, the electronic consent document managing unit 21 transmits a print request of an electronic consent document based on the electronic consent document data to the print server 6; and the print server 6 receives the print request and causes the image forming apparatus 5 to print the electronic consent document on the basis of the print request.

In this embodiment, the aforementioned signature data is raster image data of a handwriting signature. Specifically, handwriting stokes inputted with a stylus pen or the like are detected by the input device 33 (touch panel), and data of an image that includes the handwriting stokes is generated as the signature data.

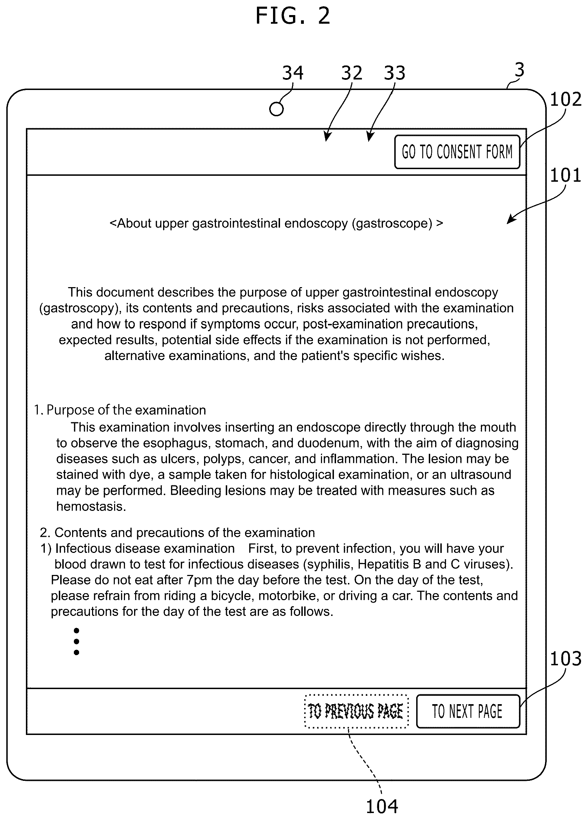

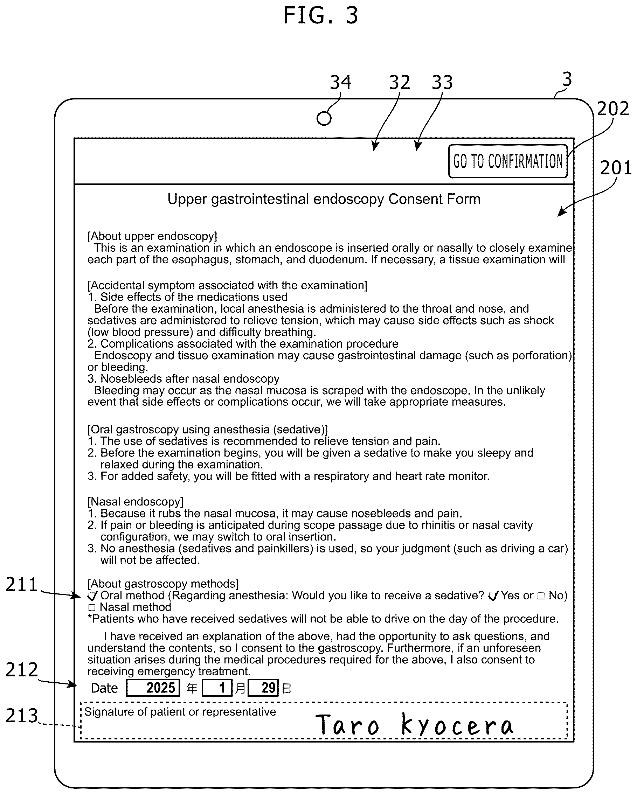

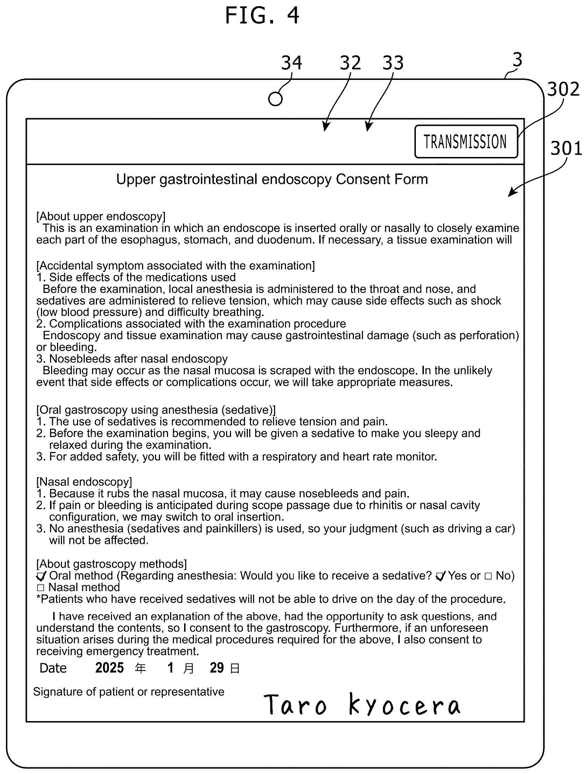

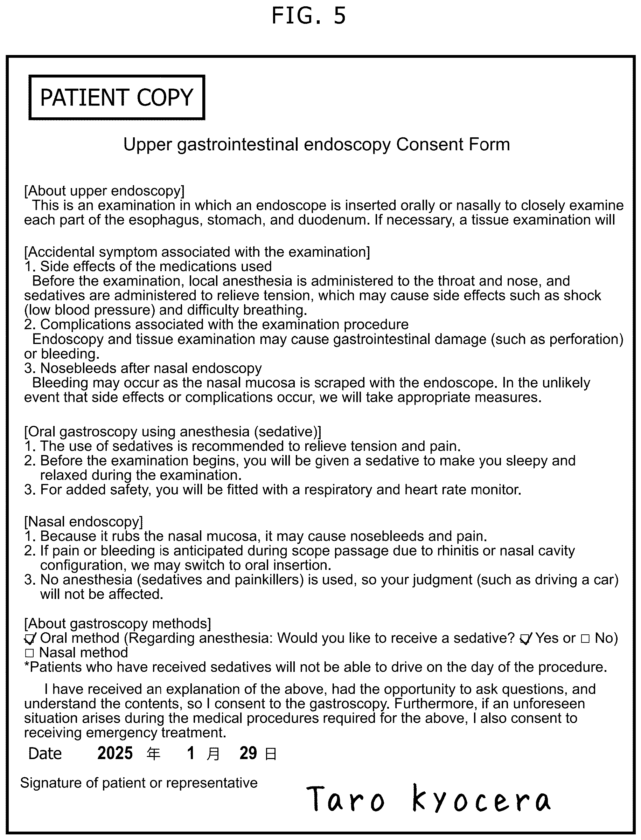

Fig. 2 shows a diagram that indicates a display example of an explanation document image. Fig. 3 shows a diagram that indicates a display example of an electronic consent form after date input and handwriting signature. Fig. 4 shows a diagram that indicates a display example of an electronic consent form (confirmation image). Fig. 5 shows a diagram that indicates an example of the printed electronic consent document.

Further, in this embodiment, the user interface processing unit 41 in the terminal apparatus 3 (a) displays an explanation document image of the aforementioned specific matter on the display device 32 as shown in Fig. 2 for example, (b) displays an electronic consent form of the specific matter after displaying the explanation document image on the display device 32 as shown in Fig. 3 for example, (c) displays an electronic consent document (i.e. confirmation image) based on the electronic consent document data after detecting the aforementioned signature operation using the input device 33 on the display device 32 as shown in Fig. 4 for example, and (d) transmits the electronic consent document data to the management server 1 using the communication device 31 after detecting a predetermined confirmation operation using the input device 33. Consequently, the signed electronic consent document is printed on a print paper sheet or the like as shown in Fig. 5, for example. The printed signed electronic consent document is handed over to a patient as a patient copy.

As shown in Fig. 2 for example, in a display screen of the explanation document image 101, soft keys 102, 103 and 104 are displayed together with the explanation document image 101. The soft key 102 is a soft key to change a screen to a display screen of the electronic consent form, the soft key 103 is a soft key to change a displayed page to a next page among plural pages in the explanation document image 101, and the soft key 104 is a soft key to change a displayed page to a previous page among plural pages in the explanation document image 101.

For example, the soft key 102 may be displayed so as to be operatable after all the pages of the explanation document image 101 are displayed, and before all the pages of the explanation document image 101 are displayed, the soft key 102 may be grayed out.

Further, when the soft key 102 is pressed down, the user interface processing unit 41 changes a screen to a display screen of the electronic consent form. As shown in Fig. 3 for example, in the display screen of the electronic consent form, the electronic consent form 201 as a document image and a soft key 202 are displayed. The soft key 202 is a soft key to change a screen to a confirmation screen of the electronic consent document.

The electronic consent form 201 includes a check box 211, a signature date input field 212, and a handwriting signature area 213. In the check box 211, a check image is displayed correspondingly to a check operation. The signature date input field 212 is pull-down lists; a year, a month and a day of the signature date are selected from the pull-down lists in accordance with user operations; and the selected year, the selected month and the selected day are displayed in the signature date input field 212. In the handwriting signature area 213, handwriting strokes of handwriting signature input are acquired, and a handwriting signature image is displayed correspondingly to the handwriting strokes.

The soft key 202 may be displayed so as to be operatable after the handwriting signature image is acquired, and therebefore the soft key 202 may be grayed out.

Further, when the soft key 202 is pressed down, the user interface processing unit 41 changes a screen to a confirmation screen of the electronic consent document. As shown in Fig. 4 for example, in the confirmation screen, an electronic consent document image 301 and a transmission key 302 are displayed. The transmission key 302 is a soft key to transmit the electronic consent document data to the management server 1. When the transmission key 302 is pressed down, the user interface processing unit 41 transmits the electronic consent document data to the management server 1 using the communication device 31.

Further, the user interface processing unit 41 in the terminal apparatus 3 (a) detects an additionally-writing operation performed by a healthcare worker onto the explanation document image while the explanation document image is displayed and generates an additionally-writing image based on the additionally-writing operation, (b) generates a signature image based on the signature operation, and (c) includes the explanation document image, the additionally-writing image, the electronic consent form, and the signature image into the electronic consent document data. In this case, for example, the additionally-writing image and the signature image are included into the electronic consent document data such that the additionally-writing image and the signature image are separated from each other (e.g. as handwriting strokes with different colors from each other).

Further, using the imaging device 34, the terminal apparatus 3 may acquire a movie of a healthcare worker in a period that the healthcare worker explains the specific matter on the basis of the explanation document image (i.e. in a period that the explanation document image is displayed), and may include movie data of the movie into the electronic consent document data. Further, a sound correction device may be installed in the terminal apparatus 3, and using the sound correction device, the terminal apparatus 3 may acquire a voice of a healthcare worker in a period that the healthcare worker explains the specific matter on the basis of the explanation document image, and may include voice data of the voice into the electronic consent document data. Consequently, at a later date, playing the movie data or the voice data in the electronic consent document data enables to verify whether the explanation was properly performed or not.

Furthermore, (a) when the aforementioned signature operation is detected, the user interface processing unit 41 in the terminal apparatus 3 may take and generate an image of a person who performs the signature operation using the imaging device 34, and (b) the user interface processing unit 41 may include the taken image into the electronic consent document data. Consequently, at a later date, displaying the taken image in the electronic consent document data enables to verify whether a person who should sign (patient or the like) properly signed or not.

Further, the user interface processing unit 41 in the terminal apparatus 3 displays the explanation document and the electronic consent form in an page order on the basis of a single file (e.g. PDF (Portable Document Format) file) generated in the page order of 1) the explanation document, 2) the electronic consent form; and the electronic consent document managing unit 21 in the management server 1 generates as an electronic consent document for storage a single file generated in a page order of 1) the electronic consent form, 2) the explanation document (i.e. in a reverse order of the displaying page order) together with the signature data on the basis of the electronic consent document data, and automatically registers the electronic consent document for storage to the electronic health record system 4 such that the electronic consent document for storage can be viewed. Consequently, the patient easily signs the electronic consent form after reading the explanation document, and at a later date, when the healthcare worker wants to confirm the signed electronic consent document, the healthcare worker can immediately see the signed electronic consent document.

Further, when the electronic consent document managing unit 21 in the management server 1 receives the electronic consent document data from any of the terminal apparatuses 3, the electronic consent document managing unit 21 may immediately transmit the print request to the print server 6 and automatically register the electronic consent document data or electronic consent document for storage (e.g. raster image data of it) based on the electronic consent document data into the electronic health record system 4.

Furthermore, at that time, after finishing printing of the electronic consent document based on the print request, the electronic consent document managing unit 21 in the management server 1 may automatically register the electronic consent document data or electronic consent document for storage based on the electronic consent document data into the electronic health record system 4.

Furthermore, when a single patient is asked to sign plural electronic consent documents of plural specific matters, the user interface processing unit 41 in the terminal apparatus 3 may display plural electronic consent forms of plural specific matters on the display device 32, detect a signature operation to a bulk signature form corresponding to the plural electronic consent forms using the input device 33, acquire signature data based on the signature operation, and transmit to the management server 1 plural pieces of the electronic consent document data corresponding to the plural electronic consent forms such that each of the plural pieces of the electronic consent document data includes the signature data. Consequently, only one signature operation is sufficient for plural electronic consent forms, and therefore, a patient convenience is gained.

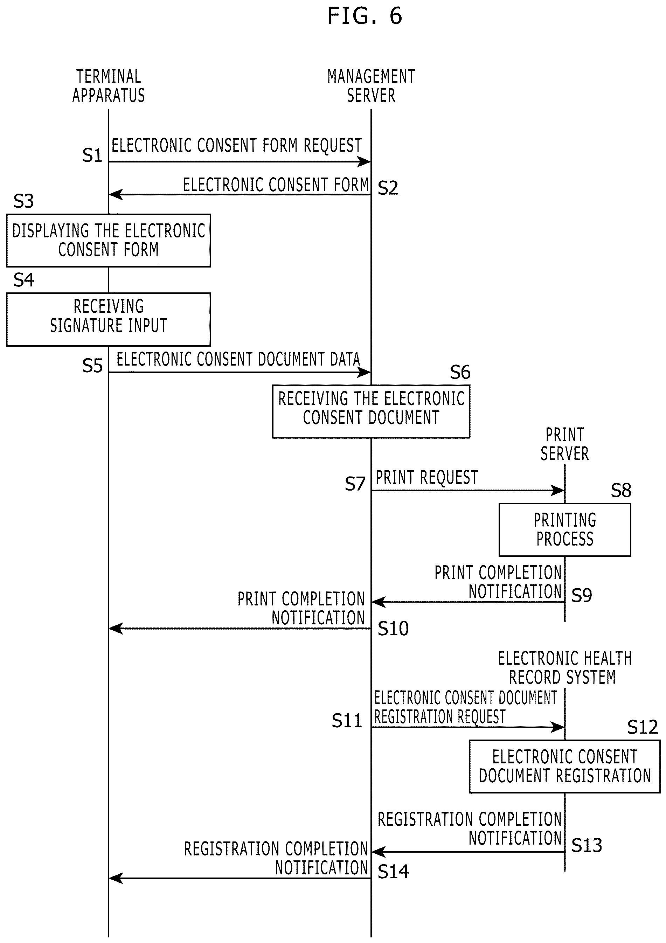

The following part explains a behavior of the aforementioned electronic consent document system. Fig. 6 shows a sequence diagram that explains a behavior of the electronic consent document system shown in Fig. 1.

When the user interface processing unit 41 in the terminal apparatus 3 detects a predetermined user operation using the input device 33, the user interface processing unit 41 transmits an electronic consent form request to the management server 1 (in Step S1). When the electronic consent document managing unit 21 in the management server 1 receives the electronic consent form request, the electronic consent document managing unit 21 transmits an electronic consent form to the terminal apparatus 3 (in Step S2).

For example, the user interface processing unit 41 includes a web client and the electronic consent document managing unit 21 includes a web server, and the user interface processing unit 41 transmits the electronic consent form request using the web client in accordance with HTTP (Hypertext Transfer Protocol). The electronic consent document managing unit 21 transmits the electronic consent form using the web server in accordance with HTTP.

When the user interface processing unit 41 in the terminal apparatus 3 receives the electronic consent form, the user interface processing unit 41 displays the electronic consent form on the display device 32 (in Step S3).

Here, as mentioned, a PDF file that includes the electronic consent form and the explanation document image is transmitted to the terminal apparatus 3, the electronic consent form and the explanation document image are displayed in turn, and after a handwriting signature operation is detected, a confirmation screen is displayed.

When an operation to the check box 211, an operation to the signature data input field 212, and a handwriting signature operation to the handwriting signature area 213 are detected by the input device 33 (in Step S4), the user interface processing unit 41 generates the electronic consent document data that includes a value of the check box 211 (i.e. checked or not), a value of the signature data input field 212 (i.e. year-month-day data), a handwriting signature image (raster image data), and the electronic consent form, and transmits the electronic consent document data (after the confirmation screen is displayed) (in Step S5).

When the electronic consent document managing unit 21 in the management server 1 receives the electronic consent document data (in Step S6), the electronic consent document managing unit 21 generates a print request of the electronic consent document and transmits the print request to the print server 6 (in Step S7). Before generating the print request, the electronic consent document managing unit 21 may determine whether the signature data is included in the electronic consent document data or not, and if the signature data is not included, may terminate the process due to error.

When the print server 6 receives the print request, the print server 6 spools the print request, and transmits the print request and thereby causes the image forming apparatus 5 to perform printing (in Step S8). In this process, the print server 6 may immediately transmit the print request to the image forming apparatus 5 in a push-printing manner and thereby causes to perform printing or may transmit the print request to the image forming apparatus 5 after receiving a request from the image forming apparatus 5 in a pull-printing manner.

When printing of the electronic consent document is finished, the print server 6 transmits a print completion notification to the management server 1 (in Step S9), and when the electronic consent document managing unit 21 in the management server 1 receives the print completion notification, the electronic consent document managing unit 21 transmits it to the terminal apparatus 3 (in Step S10). For example, when the user interface processing unit 41 in the terminal apparatus 3 receives the print completion notification, the user interface processing unit 41 displays a message that indicates print completion on the display device 32.

Further, when the electronic consent document managing unit 21 in the management server 1 receives the print completion notification, the electronic consent document managing unit 21 immediately transmits an electronic consent document registration request that includes the electronic consent document to the electronic health record system 4 (in Step S11).

When receiving the electronic consent document registration request, the electronic health record system 4 registers the electronic consent document for storage (in Step S12).

For example, the electronic consent form is acquired from the electronic health record system 4 together with a patient ID of a patient of which an electronic consent document should be acquired, and in this electronic consent document system, the electronic consent document data is acquired in association with the patient ID, and the electronic consent document data is registered in association with the patient ID in the electronic health record system 4 such that the electronic consent document data can be viewed. Consequently, in the electronic health record system 4, the electronic consent document of the patient with the patient ID can be viewed.

When finishing the registration of the electronic consent document, the electronic health record system 4 transmits a registration completion notification to the management server 1 (in Step S13), and when the electronic consent managing unit 21 in the management server 1 receives the registration completion notification, the electronic consent managing unit 21 transmits it to the terminal apparatus 3 (in Step S14). For example, when the user interface processing unit 41 in the terminal apparatus 3 receives the registration completion notification, the user interface processing unit 41 displays on the display device 32 a message that indicates registration completion of the electronic consent document to the electronic health record system 4. Further, after receiving the registration completion notification of the electronic consent document, the electronic health record system 4 or the electronic consent document managing unit 21 may immediately output an order for the aforementioned specific matter (i.e. instruction for performing the specific matter).

As mentioned, in the electronic consent system according to the aforementioned embodiment, the management server 1 and the terminal apparatus 3 are connected to the in-hospital network 2. The terminal apparatus 3 (a) displays an explanation document image of a specific matter, (b) displays an electronic consent form of the specific matter after displaying the explanation document image, (c) acquires signature data on the basis of a signature operation to the electronic consent form, (d) after detecting the signature operation, displays an electronic consent document based on electronic consent document data that includes the signature data corresponding to the electronic consent form, and (e) transmits the electronic consent document data to the management server 1 after detecting a predetermined confirmation operation. Further, the terminal apparatus 3 (a) detects an additionally-writing operation performed by a healthcare worker onto the aforementioned explanation document image and generates an additionally-writing image based on the additionally-writing operation, (b) generates a signature image based on the aforementioned signature operation, and (c) includes the explanation document image, the additionally-writing image, the electronic consent form, and the signature image into the electronic consent document data and transmits the electronic consent document data to the management server 1. Here, the additionally-writing image and the signature image are included into the electronic consent document data such that the additionally-writing image and the signature image are separated from each other.

Consequently, it can be clear that a patient's consent has been acquired after a sufficient explanation of a specific matter, and at a later date, doubts on whether the patient's consent has been obtained or not are restrained in the patient side or a third party.

It should be understood that various changes and modifications to the embodiments described herein will be apparent to those skilled in the art. Such changes and modifications may be made without departing from the spirit and scope of the present subject matter and without diminishing its intended advantages. It is therefore intended that such changes and modifications be covered by the appended claims.

For example, in the aforementioned embodiment, the check box 211 in the electronic consent form 201 may be displayed with different colors before and after being checked. Consequently, forgetting to check is restrained.

## Claims

1. An electronic consent document system, comprising:
a management server (1) connected to an in-hospital network (2); and
a terminal apparatus (3) connected to the in-hospital network (2);
wherein the terminal apparatus (3) displays an explanation document image of a specific matter; displays an electronic consent form of the specific matter after displaying the explanation document image; acquires signature data on the basis of a signature operation to the electronic consent form; after detecting the signature operation, displays an electronic consent document based on electronic consent document data that includes the signature data corresponding to the electronic consent form; and transmits the electronic consent document data to the management server (1) after detecting a predetermined confirmation operation;
the terminal apparatus (3) detects an additionally-writing operation performed by a healthcare worker onto the explanation document image and generates an additionally-writing image based on the additionally-writing operation; generates a signature image based on the signature operation; and includes the explanation document image, the additionally-writing image, the electronic consent form, and the signature image into the electronic consent document data and transmits the electronic consent document data to the management server (1); and
the additionally-writing image and the signature image are included into the electronic consent document data such that the additionally-writing image and the signature image are separated from each other.

2. The electronic consent document system according to claim 1, wherein the signature data is raster image data of a handwriting signature.

3. The electronic consent document system according to claim 1, wherein the terminal apparatus (3) comprises at least one of a sound correction device and an imaging device (34), acquires at least one of a voice and a movie of the healthcare worker in a period that the healthcare worker explains the specific matter on the basis of the explanation document image, and includes at least one of the voice and the movie into the electronic consent document data.

4. The electronic consent document system according to claim 1, wherein the terminal apparatus (3) comprises an imaging device (34), and when detecting the signature operation, takes and generates an image of a person who performs the signature operation using the imaging device and includes the taken image into the electronic consent document data.

5. The electronic consent document system according to claim 1, wherein the terminal apparatus (3) displays the explanation document and the electronic consent form on the basis of a single file generated in a page order of 1) the explanation document, 2) the electronic consent form; and
the management server (1) generates as an electronic consent document for storage a single file generated in a page order of 1) the electronic consent form, 2) the explanation document together with the signature data on the basis of the electronic consent document data, and automatically registers the electronic consent document for storage to an electronic health record system (4).

6. The electronic consent document system according to claim 1, further comprising a print server (6) connected to the in-hospital network (2);
wherein when the management server (1) receives the electronic consent document data from the terminal apparatus (3), the management server (1) transmits a print request based on the electronic consent document data to the print server (6); and
the print server (6) receives the print request, and causes an image forming apparatus (5) to print the electronic consent document on the basis of the print request.

7. The electronic consent document system according to claim 6, wherein when the management server (1) receives the electronic consent document data from the terminal apparatus (3), the management server (1) immediately transmits a print request based on the electronic consent document data to the print server (6), and automatically registers to an electronic health record system (4) either the electronic consent document data or an electronic consent document for storage based on the electronic consent document data.

8. The electronic consent document system according to claim 7, wherein after printing of the electronic consent document based on the print request is finished, the management server (1) automatically registers to an electronic health record system (4) either the electronic consent document data or an electronic consent document for storage based on the electronic consent document data.

9. The electronic consent document system according to claim 1, wherein the terminal apparatus (3) displays plural electronic consent forms of plural specific matters, acquires signature data based on a signature operation to a bulk signature form corresponding to the plural electronic consent forms, and transmits to the management server (1) plural pieces of the electronic consent document data corresponding to the plural electronic consent forms such that each of the plural pieces of the electronic consent document data includes the signature data.
